# EUROPEAN PATENT APPLICATION

(11) **EP 3 587 543 A1**
(43) Date of publication of application: **01.01.2020**
(21) Application number: 18757927.1
(22) Date of filing: 21.02.2018
(51) Int. Cl.: C11C 3/00, A23L 9/00, A61K 8/00, A61K 31/20, A61Q 19/00, C11B 7/00

(54) **SHEA OLEIN AND METHOD FOR PREPARING SAME**

(30) Priority: 23.02.2017 JP 2017032430; 22.03.2017 JP 2017055550
(71) Applicant: Fuji Oil Holdings Inc., Izumisano-shi, Osaka 598-8540 (JP)
(72) Inventor: SUZUKI, Yuta, Izumisano-shi Osaka 598-8540 (JP)
(74) Representative: Becker Kurig Straus
(86) International application number: PCT/JP2018/006209
(87) International publication number: WO 2018/155490

(57) **Abstract**

The present invention addresses the problem of providing shea olein which can maintain fluidity in a liquid state for a long time at ambient temperature, and in a preferred embodiment, can maintain fluidity in a liquid state for a long time over even a wide temperature range from low temperature to ambient temperature. The problem can be solved by preparing shea olein containing adjusted amounts of constituent components.

## Description

### [Technical Field]

The present invention relates to shea olein and a method for preparing the same. More specifically, the present invention relates to shea olein which can maintain fluidity in a liquid state for a long time at ambient temperature, and in a preferred embodiment, can maintain fluidity in a liquid state for a long time over a wide temperature range from low temperature to ambient temperature; and a method for preparing the same.

### [Background Art]

Shea butter is a generic term for oils and fats obtained from the seeds of shea trees (Butyrospermum parkii) which inhabit a region commonly known as the Shea belt in North Central Africa. Shea butter is in a solid state at ambient temperature and may be used while its physical properties are maintained. But in many cases, shea butter is used after fractionating into a solid part and a liquid part thereof in order to enhance the functionality of the solid part as a substitute oil or fat for cacao butter. In addition to use as a substitute oil or fat for cacao butter, utilizing its excellent characteristics, the solid side obtained by fractionation is widely used in chocolate products, and is often used for the purpose of improving the functionality of chocolates, such as prevention of a so-called fat bloom, which is a phenomenon in which the surface of chocolate products becomes whitened, or the inside thereof becomes powdered (Patent Literature 1 to 3).

The liquid part called shea olein which is obtained by fractionation has been studied for use as a cosmetic raw material. It is also used as a raw material for many chemical products in the Japanese market, and demand in its non-food applications are also expanding. In addition, Patent Literature 4 reports that a raw material derived from shea butter is suitable for cosmetics.

### [Reference List]

### [Patent Literature]

[Patent Literature 1]
   Japanese Unexamined Patent Application Publication No. S52-063206
[Patent Literature 2]
   Published Japanese Translation No. 2015-533482 of the PCT International Publication
[Patent Literature 3]
   Japanese Unexamined Patent Application Publication No. 2001-98293
[Patent Literature 4]
   Japanese Unexamined Patent Application Publication No. 2011-132207
[Patent Literature 5]
   Japanese Unexamined Patent Application Publication No. 2013-189398 [Patent Literature 6]
   Japanese Examined Patent Application Publication No. H06-87787

### [Summary of Invention]

### [Technical Problem]

Development of applications of shea olein which is the liquid side cannot be said to have advanced as compared with those of shea stearin of the solid side obtained by fractionating shea butter. The reasons why the development of applications has not progressed will be considered.

Regarding applications in chemical products, relatively large amounts of oil are used for cleansing oils and massage oils. Cleansing oils are manufactured and sold mainly for the purpose of removing dirt components and cosmetic components from the skin, and the condition in which this oil is in a liquid state and has fluidity at ambient temperature is important from the viewpoint of handling and use sensation. In addition, cleansing oils have widely spread all over the world recently, and the problem of freezing and solidification of cleansing oils has been reported (Patent Literature 5). Regarding this, it has been reported that once a cleansing oil is frozen, separation and precipitation of components occur even when it is remelted, and this may impair the use sensation and functionality thereof. Furthermore, massage oils are also desirably in a liquid state at ambient temperature, and the same physical properties are required for a raw material oil.

In response to the above demand, when shea butter in a liquid state which is currently available on the market and the fractionated liquid side of Patent Literature 1 are left at low temperature, use of them as a raw material for cleansing oils and massage oils is difficult. This makes it clear that various problems occur when shea olein is used for such a purpose.

Regarding applications in food products, salad oils and coffee creamers which are oil food raw materials in a liquid state at ambient temperature are respectively used for frying foods and imparting a suitable flavor and mildness to coffee. Meanwhile, oils and fats are present in a liquid state and a solid state, and those in which crystals are precipitated at ambient temperature or those solidify at ambient temperature are not suitable for the above-mentioned food products. For example, even if a liquid state is temporarily maintained, when crystals are precipitated as time passes, the quality and value of products are greatly reduced. Accordingly, oils and fats used for these food products are required to be maintained in a liquid state at ambient temperature as much as possible. In response to such a demand, studies as described in Patent Literature 6 in which a palm oil is used as a raw material have been performed.

Meanwhile, when shea butter in a liquid state which is currently available on the market and the fractionate liquid side of Patent Literature 1 are left at low temperature, they lose fluidity, and therefore use of them as a raw material for cleansing oils and massage oils is difficult. This makes it clear that various problems occur when shea olein is used for such a purpose.

In order to satisfy the above-described demands, an object of the present invention is to provide shea olein which can maintain fluidity in a liquid state for a long time at ambient temperature, and in a preferred embodiment, can maintain fluidity in a liquid state for a long time over a wide temperature range from low temperature to ambient temperature; and is to expand an amount of shea olein used.

### [Solution to Problem]

In order to achieve the aforementioned objects, as a result of intensive studies, the inventors of the present invention have found that the above-mentioned problems can be solved by adjusting a content of components constituting shea olein, and therefore have completed the present invention.

In other words, the present invention is as follows.
(1) Shea olein which satisfies all of the following (A) to (C):
   (A) An iodine value is 73 or more,
   (B) StOSt is 2.0% by weight or less, and
   (C) A content of diacylglycerol (DG) is 10% by weight or less,
   where St represents stearic acid and O represents oleic acid.
(2) The shea olein according to (1), in which a content of diacylglycerol (DG) is 6.5% by weight or less.
(3) The shea olein according to (2), which satisfies all of the following (A) to (D):
   (A) An iodine value is 80 or more,
   (B) StOSt is 1.0% by weight or less,
   (C) StOO is 20.0% by weight or less, and
   (D) A content of diacylglycerol (DG) is 3.0% by weight or less.
(4) The shea olein according to (1), which is in a clear state after being stored at 15°C for 7 days in the following oil and fat storage test,
   in which in a method of the oil and fat storage test,
   approximately 5 g of oils and fats completely melted at 80°C is weighed into a test tube, and
   the weighed test tube is stored at 60°C for 1 hour, and then is stored at 15°C.
(5) The shea olein according to (2), which is in a clear state after being stored at 5°C for 6 hours in the following oil and fat storage test,
   in which in a method of the oil and fat storage test,
   approximately 5 g of oils and fats completely melted at 80°C is weighed into a test tube, and
   the weighed test tube is stored at 60°C for 1 hour, and then is stored at 5°C.
(6) The shea olein according to (3), which is in a clear state after being stored at 5°C for 4 days in the following oil and fat storage test,
   in which in a method of the oil and fat storage test,
   approximately 5 g of oils and fats completely melted at 80°C is weighed into a test tube, and
   the weighed test tube is stored at 60°C for 1 hour, and then is stored at 5°C.
(7) A chemical product which is formed of the shea olein according to any one of (1) to (6).
(8) The chemical product according to (7), which is a cleansing oil or a massage oil.
(9) A food product which is formed of the shea olein according to any one of (1) to (6).
(10) The food product according to (9), which is a salad oil or a coffee creamer.
(11) A method for preparing shea olein, including: using shea olein having an iodine value of 60 to 72 as a raw material; and using hexane as a fractionating solvent.
(12) A method for preparing shea olein, including: using shea butter having an iodine value of 50 to 65 or shea olein having an iodine value of 60 to 72 as a raw material; and using acetone as a fractionating solvent.
(13) A method for preparing shea olein, including: reducing an amount of DG by chemical treatment or enzymatic treatment using she olein obtained in (12) as a raw material.

### [Advantageous Effects of Invention]

According to the present invention, it is possible to obtain shea olein which can maintain fluidity in a liquid state for a long time at ambient temperature.

As a preferred embodiment, it is possible to extend a flowable time in a liquid state at ambient temperature as compared to currently available shea olein. As a more preferred embodiment, it is possible to extend a flowable time in a liquid state over a wide temperature range from low temperature to ambient temperature.

In addition, as an even more preferred embodiment, according to the present invention, it is possible to expand usage applications of shea olein in chemical products and food products.

### [Description of Embodiments]

Hereinafter, the present invention will be described in more detail.

Shea olein of the present invention is a low melting point component obtained from shea olein or shea butter and is a shea olein satisfying the following numerical value ranges.

The shea olein of the present invention is a shea olein satisfying all of the following (A) to (C):
(A) an iodine value is 73 or more.
(B) StOSt is 2.0% by weight or less.
(C) a content of diacylglycerol (hereinafter referred to as DG) is 10% by weight or less.

A content of DG is preferably 6.5% by weight or less. It is then possible to extend a flowable time in a liquid state at low temperature.

The shea olein of the present invention is preferably shea olein satisfying all of the following (A) to (D). This is preferable because cold resistance is then improved.
(A) An iodine value is 80 or more.
(B) StOSt is 1.0% by weight or less.
(C) StOO is 20.0% by weight or less.
(D) A content of DG is 3.0% by weight or less.

A more preferred embodiment of the shea olein of the present invention is shea olein satisfying at least one of the following (A) to (D). This is preferable because cold resistance further is then improved.
(A) An iodine value is 85 or more.
(B) StOSt is 0.5% by weight or less.
(C) StOO is 10.0% by weight or less.
(D) A content of DG is 2.0% by weight or less.

An even more preferred embodiment of the shea olein of the present invention is shea olein satisfying all of the following (A) to (D). This is preferable because cold resistance is then improved even further.
(A) An iodine value is 85 or more.
(B) StOSt is 0.5% by weight or less.
(C) StOO is 10.0% by weight or less.
(D) A content of DG is 2.0% by weight or less.

A method for preparing the shea olein of the present invention is not limited as long as shea olein or shea butter is used as a raw material and the above-described numerical value range is satisfied, but it is preferable to use shea olein which is a liquid side obtained after a solid part is obtained as a substitute oil or fat for cacao butter. This is because new added value can be given to this product for which the present usage applications are limited, and which are being sold as for feed or cheap frying oil.

The shea olein of the present invention can also be prepared directly from shea butter rather than shea olein, and any shea butter can be used regardless of its origin or production region as long as shea butter is derived from shea trees.

Examples of the method for preparing shea olein of the present invention include solvent fractionation, dry fractionation, detergent fractionation, and the like, but from the viewpoint of a quality which is obtained, hexane solvent fractionation is preferable, and acetone solvent fractionation is more preferable. A degumming process is carried out to remove unsaponifiable matter contained in raw material shea butter, but any method can be adopted as long as unsaponifiable matter can be removed and thereby the amount thereof reduced.

In addition, as a preferred embodiment, it is preferable to reduce a DG content by a known DG treatment method because cold resistance can be improved. This method is preferably carried out after acetone fractionation, and a DG content can be reduced by chemical treatment or enzymatic treatment. Examples of the chemical treatment include contact treatment using an adsorbent such as silica gel, column treatment, and alkaline decomposition. Examples of the enzymatic decomposition include methods using a lipase enzyme.

A solvent fractionation method using hexane will be illustrated. Melted shea olein and hexane are mixed to become 3:7, a temperature is gradually lowered while stirring weakly, and when the temperature reaches -20°C, the temperature is kept constant for about 30 minutes. The obtained mixed solution is fractionated into a solid part and a liquid part by filtering, and the shea olein of the present invention is obtained as the obtained liquid part.

A solvent fractionation method using acetone will be illustrated. Melted shea olein and acetone are mixed to become 2:8, a temperature is gradually lowered while stirring weakly, and when the temperature reaches -10°C, the temperature is kept constant for about 30 minutes. The obtained mixed solution is fractionated into a solid part and a liquid part by filtering, and the shea olein of the present invention is obtained as the obtained liquid part.

A DG treatment method will be illustrated. After performing acetone fractionation, 0.5 mL of about 20 wt% NaOH aqueous solution is mixed with 1 kg of the obtained liquid part and stirred to decompose DG, and then neutralization and washing with water are performed to remove alkalis, glycerin, and free fatty acids.

The shea olein of the present invention can be used in combination with other oils and fats. Usable oil and fat types are not particularly limited, and examples thereof include vegetable oils and fats such as rapeseed oil, soybean oil, sunflower seed oil, cottonseed oil, peanut oil, rice bran oil, corn oil, safflower oil, olive oil, kapok oil, sesame oil, evening primrose oil, palm oil, palm kernel oil, coconut oil, medium chain triglycerides (MCT), and sal fat; animal oils and fats such as milk fat, beef fat, lard, fish oil, and whale oil; hardened oils, fractionated oils, hardened and fractionated oils, fractionated and hardened oils, and processed oils and fats subjected to ester exchange and the like with these oils; mixed oils and fats thereof; and the like. Preferred examples are rapeseed oil, soybean oil, sunflower seed oil, cottonseed oil, peanut oil, rice bran oil, corn oil, safflower oil, and olive oil which are in a liquid state at ambient temperature, and more preferred examples are sunflower seed oil and olive oil.

A preferred embodiment of the shea olein of the present invention, is shea olein that is obtained in a clear state after being stored at 15°C for 7 days in the following oil and fat storage test.

### (Method of oil and fat storage test)

- Approximately 5 g of oils and fats completely melted at 80°C is weighed into a test tube.
- The weighed test tube is stored at 60°C for 1 hour, and then is stored at 15°C.

As a more preferred embodiment of the shea olein of the present invention, shea olein that is in a clear state after being stored at 5°C for 6 hours in the following oil and fat storage test is obtained when a content of diacylglycerol (DG) is 6.5% by weight or less.

### (Method of oil and fat storage test)

- Approximately 5 g of oils and fats completely melted at 80°C is weighed into a test tube.
- The weighed test tube is stored at 60°C for 1 hour, and then is stored at 5°C.

As an even more preferable embodiment, shea olein that is in a clear state after being stored at 5°C for 4 days in the following oil and fat storage test is obtained when shea olein satisfies all of the following (A) to (D).
(A) An iodine value is 80 or more.
(B) StOSt is 1.0% by weight or less.
(C) StOO is 20.0% by weight or less.
(D) A content of diacylglycerol (DG) is 3.0% by weight or less.

The shea olein of the present invention can be used for chemical products. Examples of chemical products include shampoos, body soaps, and hand creams. Preferred usage application is application that requires clarity, and examples thereof include liquid lotions, sunscreens, and perfumes which are in a settled liquid state at ambient temperature. More preferred usage application is cleansing oils or massage oils.

When used for chemical products, optional components such as colorants, surfactants, and antioxidants which are used in general chemical products can be appropriately added to the shea olein of the present invention. An amount added thereof is 20% by weight or less and is preferably 10% by weight or less with respect to the shea olein of the present invention. These may be used in a combination of 2 or more kinds thereof.

The shea olein of the present invention can be used for foods. Preferred foods for which it can be used are chocolates and cookies. More preferred usage application is application that requires clarity, and examples thereof include salad oils and coffee creamers which are in a settled liquid state at ambient temperature.

When used for foods, optional components such as colorants, surfactants, antioxidants, and crystal inhibitors which are used in general food products can be appropriately added to the shea olein of the present invention. An amount added thereof is 20% by weight or less and is preferably 10% by weight or less with respect to the shea olein of the present invention. 5% by weight or less is more preferable, and 3% by weight or less is even more preferable. These may be used in combination of 2 or more kinds thereof.

At present, most shea butters in a liquid state sold on the market have an iodine value of about 72, and a main triglyceride composition thereof is a composition in which StOSt is 2.5% or more, StOO is about 35%, and DG is 6.5% or more. In a preferred embodiment, a flowable time of the shea olein of the present invention in a liquid state can be extended as compared with products of the related art by the above-described composition being used.

In the present specification, low temperature means 10°C or less, and a shea olein having cold resistance after 5°C storage is preferable as an indicator of shea olein having cold resistance at low temperature. In addition, in the present specification, an ambient temperature is 15°C to 25°C, and shea olein having storage resistance at 15°C storage is preferable as an indicator of shea olein having storage resistance at ambient temperature. Evaluation indicators for cold resistance and storage resistance will be defined in the examples, but as an indicator of shea olein that can withstand distribution and use conditions in a liquid state, shea olein that does not separate and has fluidity even if crystals are slightly precipitated is considered as a pass. Shea olein that is in a semitransparent state but in a state in which crystals are not precipitated is preferable, and shea olein that can be maintained in a clear state without crystal precipitation is more preferable.

The shea olein of the present invention is an invention which finds new added value of shea olein that is a low-value-added oil obtained when fractionating shea butter obtained from seeds of shea trees, and which helps effective use of shea olein.

In addition, in usage applications in chemical products, it is an invention which helps effective use of triterpene esters (an anti-inflammatory effect, a UV absorbing effect) specifically contained in the present oil and fat.

### [Examples]

Hereinafter, the present invention will be described in more detail by illustrating examples of the present invention. In the examples, both % and parts mean on a weight basis.

### (Degumming method)

Shea butter and acetone were mixed at 20:80, and the gum was precipitated while stirring at 27°C for 30 minutes. The mixture was allowed to stand and the sedimented gum was filtered off to obtain degummed shea butter.

### (Hexane fractionation method)

Melted shea olein and hexane were mixed to become 3:7, a temperature was gradually lowered while stirring weakly, and when the temperature reached a set fractionation temperature, the temperature was kept constant for about 30 minutes. The obtained mixed solution was fractionated into a solid part and a liquid part by filtering, and the shea olein of the present invention was obtained as the obtained liquid part.

### (Acetone separation method)

Melted shea olein and acetone were mixed to become 2:8 (18:82 in Comparative Example 1), a temperature was gradually lowered while stirring weakly, and when the temperature reached a set fractionation temperature, the temperature was kept constant for about 30 minutes. The obtained mixed solution was fractionated into a solid part and a liquid part by filtering, and the shea olein of the present invention was obtained as the obtained liquid part.

### (DG removal method)

0.5 mL of about 20 wt% NaOH aqueous solution was mixed with 1 kg of the obtained liquid part and stirred to decompose DG, and then neutralization and washing with water were performed.

### (Production of shea olein)

A solvent separation was performed according to the fractionation method under the conditions shown in Table 2. As a raw material shea butter, shea butter after degumming according to the above-described (Degumming method) was used, and an iodine value was 55.3. DG removal was performed according to (DG removal method). Winterization of Comparative Example 2 was maintained at 15°C for 2 days, and the precipitated solid fat was filtered off to obtain shea olein of Comparative Example 4.

### (Evaluation method)

An iodine value, HPLC, and GC were used to analyze a component composition and a TG composition. In the storage test, after melting at 80°C, about 5 g of each oil and fat was weighed into a test tube, stabilized at 60°C for 1 hour, and stored in a constant-temperature tank at 0, 5, 10 and 15°C. Evaluation was performed by regularly observing a degree of solidification according to evaluation standards of Table 1. All evaluation results are shown in Table 2. Analysis by HPLC was performed by the following method.

### (Analytical method of triglyceride composition by HPLC)

Measurement was performed on a sum of symmetrical and asymmetrical triglycerides (for example, a sum of an StStO content and an StOSt content) by high-performance liquid chromatography analysis. Measurement was performed under measurement conditions as follows (column: ODS, eluent: acetone/acetonitrile = 80/20, liquid volume: 0.9 ml/min, column temperature: 25°C, and detector: differential refractometer).

In Table 2, S is saturated fatty acids and U is unsaturated fatty acids.

In addition, USM stands for unsaponifiable material and means unsaponifiable matter.

**[Table 1]**

| | |
|---|---|
| A | No crystal precipitation and clear state |
| B | No crystallization but semi-transparent state |
| C | Slight crystallization, no liquid fractionation, and fluidity |
| D | Crystallization throughout and no fluidity |
| E | Large amounts of sediment and solidification in progress |
| F | Completely solidified |

**[Table 2]**

| | Test name | Example 1 | Example 2 | Example 3 | Example 4 | Comparative Example 1 | Comparative Example 2 | Comparative Example 3 | On-market product |
|---|---|---|---|---|---|---|---|---|---|
| Conditions | Fractionation raw material | She olein H | Shea butter | She olein H | Shea butter | Shea butter | She olein H | She olein H | Shea butter |
| | Preparation method | Hexane fractionation | Acetone fractionation | Acetone fractionation | Acetone fractionation | Acetone fractionation | Winterization | Acetone fractionation | Unclear |
| | OC (%) | 30 | 20 | 20 | 20 | 18 | 100 | 20 | Unclear |
| | Temperature (°C) | -20 | -10 | -10 | -10 | 15 | 15 | -10 | Unclear |
| | DG removal method | × | ○ | ○ | × | × | × | × | Unclear |
| IV | Liquid side | 75.6 She olein A | 91.8 She olein B | 88.9 She olein C | 91.8 She olein D | 69.3 She olein H | 70 She olein I | 83.3 She olein J | 72 On-market product |
| | Solid side | 42.8 | 47.7 | 56.5 | 47.7 | 33.4 | 67.2 | 58.2 | Unclear |
| TG composition | S3 | 0.2 | 0.8 | 0.1 | 0.7 | 0.5 | 0.3 | 0.5 | 0.2 |
| | S2U | 11.3 | 6 | 7.7 | 5.4 | 19.9 | 19.9 | 8.5 | 17.5 |
| | StOSt | 1.5 | 0.1 | 0.3 | 0.1 | 6.4 | 6.2 | 0.8 | 2.8 |
| | SU2 | 55.4 | 27.6 | 26.2 | 24.6 | 46.8 | 48 | 36 | 51.7 |
| | StOO | 39.7 | 7.3 | 7.1 | 6.7 | 34.2 | 34.9 | 20.1 | 36.6 |
| | U3 | 9.6 | 20.9 | 20.6 | 18.4 | 7.8 | 7.9 | 13.2 | 8.4 |
| Components | USM | 12.1 | 35.2 | 29.9 | 30.3 | 8.1 | 8.6 | 16.5 | 10.8 |
| | DG | 6.1 | 1.2 | 1.2 | 9.4 | 7.5 | 6.5 | 11.8 | 6.8 |
| | TG | 81.5 | 54.3 | 68.5 | 58.1 | 82.3 | 85 | 71.7 | 82.3 |
| Storage test result | 0°C/6 hr | B | A | A | B | E | D | C | E |
| | 5°C/6hr | A | A | A | B | E | D | C | D |
| | 10°C/6hr | A | A | A | A | C | A | C | A |
| | 15°C/6hr | A | A | A | A | C | A | A | A |
| | 0°C/D+4 | D | A | A | B | F | F | F | F |
| | 5°C/D+4 | C | A | A | D | F | F | D | D |
| | 10°C/D+4 | A | A | A | D | F | B | D | D |
| | 15°C/D+4 | A | A | A | A | D | A | D | C |
| | 0°C/D+7 | F | A | B | B | F | F | F | F |
| | 5°C/D+7 | D | A | A | D | F | F | F | F |
| | 10°C/D+7 | D | A | A | D | F | F | E | F |
| | 15°C/D+4 | A | A | A | A | E | A | E | D |
| Comprehensive evaluation | | B | A | A | B | D | D | D | C |

| | | | | | | | | | |
|---|---|---|---|---|---|---|---|---|---|
| * OC (%) indicates a content of oils and fats, and TG composition and Component indicates % by weight. | | | | | | | | | |

### (Conclusions)

- As storage test results, the shea oleins A to D obtained in Examples 1 to 4 were in a clear state even after being stored at 15°C for 7 days.
- As storage test results, the shea oleins A to C obtained in Examples 1 to 3 were in a clear state even after being stored at 5°C for 6 hours.
- As storage test results, the shea oleins A to C obtained in Examples 1 to 3 were in a liquid state even after being stored at 5°C for 4 days and a flowable state was maintained. In addition, the shea oleins B and C of Example 2 and Example 3 were in a clear state even after being stored for 7 days.
- The shea oleins B and C of Example 2 and Example 3 were in a clear state even after being stored at 0°C for 4 days.
- The shea olein of Comparative Example 2 could maintained in a clear state when being stored at 15°C but did not pass the test, because when at 10°C that is a slightly low temperature, storage resistance lowered significantly, and productivity was poor.

### [Industrial Applicability]

According to the present invention, by devising the fractionation method, it is possible to provide shear olein having strong cold resistance. According to the present invention, it is possible to expand usage applications of shea olein for which liquid fluidity is required.

## Claims

1. A shea olein which satisfies all of the following (A) to (C):
(A) an iodine value is 73 or more,
(B) StOSt is 2.0% by weight or less, and
(C) a content of diacylglycerol (DG) is 10% by weight or less,
where St represents stearic acid and O represents oleic acid.

2. The shea olein according to claim 1, wherein the content of diacylglycerol (DG) is 6.5% by weight or less.

3. The shea olein according to claim 2, which satisfies all of the following (A) to (D):
(A) an iodine value is 80 or more,
(B) StOSt is 1.0% by weight or less,
(C) StOO is 20.0% by weight or less, and
(D) a content of diacylglycerol (DG) is 3.0% by weight or less.

4. The shea olein according to claim 1, which is in a clear state after being stored at 15°C for 7 days in the following oil and fat storage test,
wherein in a method of oil and fat storage test,
approximately 5 g of oils and fats completely melted at 80°C is weighed into a test tube, and
the weighed test tube is stored at 60°C for 1 hour, and then is stored at 15°C.

5. The shea olein according to claim 2, which is in a clear state after being stored at 5°C for 6 hours in the following oil and fat storage test,
wherein in a method of oil and fat storage test,
approximately 5 g of oils and fats completely melted at 80°C is weighed into a test tube, and
the weighed test tube is stored at 60°C for 1 hour, and then is stored at 5°C.

6. The shea olein according to claim 3, which is in a clear state after being stored at 5°C for 4 days in the following oil and fat storage test,
wherein in a method of oil and fat storage test,
approximately 5 g of oils and fats completely melted at 80°C is weighed into a test tube, and
the weighed test tube is stored at 60°C for 1 hour, and then is stored at 5°C.

7. A chemical product which is formed of the shea olein according to any one of claims 1 to 6.

8. The chemical product according to claim 7, which is a cleansing oil or a massage oil.

9. A food product which is formed of the shea olein according to any one of claims 1 to 6.

10. The food product according to claim 9, which is a salad oil or a coffee creamer.

11. A method for preparing shea olein, comprising:
using shea olein having an iodine value of 60 to 72 as a raw material; and
using hexane as a fractionating solvent.

12. A method for preparing shea olein, comprising:
using shea butter having an iodine value of 50 to 65 or shea olein having an iodine value of 60 to 72 as a raw material; and
using acetone as a fractionating solvent.

13. A method for preparing shea olein, comprising:
reducing an amount of diacylglycerol by chemical treatment or enzymatic treatment using she olein obtained in claim 12 as a raw material.
